# EUROPÄISCHE PATENTSCHRIFT

(11) **EP 0 455 049 B1**
(45) Veröffentlichungstag und Bekanntmachung des Hinweises auf die Patenterteilung: **04.01.1995**
(21) Anmeldenummer: 91106158.8
(22) Anmeldetag: 17.04.1991
(51) Int. Cl.: A61M 16/22

(54) **Verfahren zur Wiederverwendung von Anästhesiegasen bei der Inhalationsanästhesie und Anordnung zur Durchführung des Verfahrens**
Method for reuse of anaesthetics and a device for realization of the method
Procédé pour la réutilisation d'un anesthésique et dispositif pour la réalisation de ce procédé

(30) Priorität: 03.05.1990 SE 9001581
(43) Veröffentlichungstag der Anmeldung: 06.11.1991
(73) Patentinhaber: Siemens-Elema AB, 171 95 Solna 1 (SE); SIEMENS AKTIENGESELLSCHAFT, 80333 München (DE)
(72) Erfinder: Psaros, Georgios, S-146 00 Tullinge (SE); Olsson, Sven-Gunnar, S-232 59 Arlöv (SE)

(56) Entgegenhaltungen:
- WO-A-88/07876
- SE-C- 167 364
- US-A- 3 713 440
- US-A- 4 150 670
- US-A- 4 360 018

## Beschreibung

Die Erfindung bezieht sich auf ein Verfahren zur Wiederverwendung von Anästhesiegasen bei der Inhalationsanästhesie und Anordnung zur Durchführung des Verfahrens.

Durch die SE-AS 459 155 ist bekannt, daß frisches Gas mit Anästhesiemittel aus einem Anästhesiemittelvergaser in der zum Patienten ein- und ausgehenden Sammelleitung gemischt wird und daß bei der Ausatmung des Patienten das Ausatmungsgas ein Adsorptionsfilter passiert. Dasjenige Anästhesiemittel, das vom Patienten nicht absorbiert wird, wird vom Adsorptionsmaterial im Adsorptionsfilter adsorbiert, während der größte Teil des Ausatmungsgases das Filter passiert. Bei der Einatmung des Patienten wird das adsorbierte Anästhesiemittel aus dem Adsorptionsmaterial desorbiert und dem Patienten wieder zugeführt. Es kann jedoch nicht vermieden werden, daß bei solchen Adsorbtionsfiltern eine geringe Menge Kohlendioxyd aus dem Ausatmungsgas des Patienten im Adsorptionsmaterial adsorbiert, danach desorbiert und während der Einatmungsphase dem Patienten wieder zugeführt wird.

Der Erfindung liegt die Aufgabe zugrunde, ein verfahren und eine Anordnung zur Durchführung des Verfahrens der eingangs genannten Art zu schaffen, wodurch mit einfachen Mitteln verhindert wird, daß das aus dem Ausatmungsgas des Patienten im Adsorptionsmaterial adsorbierte Kohlendioxyd bei der nächsten Einatmungsphase dem Patienten zugeführt wird.

Diese Aufgabe ist durch die Merkmale des Anspruchs 1 gelöst. Dadurch, daß wenigstens das Gas, das das Adsorptionsfilter passiert hat, einen Absorber für Kohlendioxyd passiert, bevor es dem Patienten zugeführt wird, ist die Gasmischung frei von Kohlendioxyd, wenn sie den Patienten erreicht. Der Absorber kann verhältnismäßig klein gemacht werden, da er in der Einatmungsphase lediglich eine geringe Menge Kohlendioxyd aufzunehmen braucht.

Es ist nicht sinnvoll, einen Kohlendioxydabsorber in der gemeinsamen Leitung zwischen dem Adsorptionsfilter und dem Patienten in der Anordnung anzubringen, wie es in der SE-AS 459 155 beschrieben ist. Ein solcher Absorber müßte sehr groß sein um das gesamte Kohlendioxyd aus dem Ausatmungsgas des Patienten zu absorbieren. Der Absorber würde auch aufgrund seiner Größe einen unannehmbar großen Ausatmungswiderstand bilden.

In einer Weiterbildung des Verfahrens wird vorgeschlagen, daß die Gaskonzentration einer gewünschten Gassorte zwischen dem Patienten und dem Absorber gemessen und zur Steuerung des frischen Atmungs- bzw. Anästhesiegases das der Gasmischung zugeführt wird, benutzt wird. Hierdurch wird erreicht, daß der Patient immer eine gewünschte Gaskonzentration erhält.

In einer vorteilhaften Ausbildung des Verfahrens wird vorgeschlagen, daß die Steuerung Feedback-kontrolliert erfolgt, wobei die gemessene Gaskonzentration den Istwert bildet und die gewünschte Gaskonzentration den Sollwert bestimmt. Durch die erwähnte Steuerung erfolgt automatisch eine Regelung der Gaskonzentration, die dem Patienten zugeführt wird.

Nach der Erfindung wird auch eine Anordnung zur Durchführung des Verfahrens geschaffen, die eine gemeinsame Leitung für die Zufuhr und Abgabe von Anästhesie- und Atmungsgasen zu bzw. von einem Patienten umfaßt, wobei in dieser Leitung ein Adsorptionsfilter angeordnet ist, das ein Adsorptionsmaterial für die Adsorption und Desorption von Anästhesiegasen beinhaltet, und die eine zweite Leitung für die Zufuhr von Anästhesie- und/oder Atmungsgasen zum Patienten vorbei an dem Adsorptionsfilter umfaßt. Das Wesentliche dieser Anordnung ist, daß die gemeinsame Leitung zwischen dem Patienten und dem Adsorptionsfilter mindestens teilweise in einen Ein- bzw. Ausatmungszweig aufgeteilt ist, wobei in dem Einatmungszweig ein Absorber für Kohlendioxyd angeordnet ist. Durch diesen Absorber, der die Kohlendioxydreste absorbiert, wird dem Patienten in der Einatmungsphase kein Kohlendioxyd zugeführt.

In einer weiteren vorteilhaften Ausbildung der Erfindung wird vorgeschlagen, daß als Adsorptionsmaterial Kohle verwendet wird. Kohle ist ein sehr gutes Adsorptionsmaterial für Anästhesiemittel wie z.B. Halothan. Kohle ist auch ein sehr guter Feuchtigkeitsadsorber und stellt hierdurch einen guten Feuchtigkeit-Wärme-Wechsler dar. Wegen der hohen Adsorptionsfähigkeit der Kohle kann das Adsorptionsfilter verhältnismäßig klein gemacht werden.

Im Hinblick auf eine günstige konstruktive Ausgestaltung der Erfindung wird vorgeschlagen, daß das Adsorptionsfilter und der Absorber hauptsächlich paralell und mit Abstand zueinander derart in einem Behälter angebracht sind, daß ein erster Kanal zwischen dem Adsorptionsfilter und dem Absorber gebildet ist, daß ein zweiter Kanal zwischen dem Adsorptionsfilter und einer ersten Innenwand des Behälters gebildet ist und daß ein dritter Kanal zwischen dem Absorber und einer zweiten Innenwand des Behälters gebildet ist, daß der erste Kanal an dem Ausatmungszweig, der zweite Kanal an der dem Patienten abgewandten Seite des Adsorptionsfilters an der gemeinsamen Leitung und der dritte Kanal an dem Einatmungszweig angeschlossen ist. Hierdurch wird ein kleines, einfaches, billiges und kompaktes Teil erhalten, das sowohl das Adsorptionsfilter als auch den Absorber beinhaltet. Dieses Teil kann daher als Wegwerfprodukt verwendet werden.

Eine günstige Ausgestaltung der Erfindung wird dadurch erhalten, daß im Anschluß an das Adsorptionsfilter an der dem Patienten abgewandten Seite ein Keimfilter angeordnet ist. Hierdurch wird erreicht, daß Keime aus dem Ausatmungsgas herausgefiltert werden. Auf dieser Weise wird verhindert, daß Leitungen und Beatmungsgerät kontaminiert werden. Diese brauchen daher nach einer Behandlung nicht gesäubert zu werden.

Eine weitere günstige Ausgestaltung der Erfindung wird erhalten, indem an der gemeinsamen Leitung bzw. an dem Einatmungszweig zwischen dem Patienten und dem Absorber eine Gasleitung angeschlossen ist, die einen Teil der Gasmischung, die dem Patienten zugeführt wird, zu einer Vorrichtung zur Messung einer Gaskonzentration mindestens einer Gassorte führt, und wobei ein der Gaskonzentration entsprechendes Sensorsignal einer Steuervorrichtung zugeführt wird, die Feedback-kontrolliert die Zufuhr von Anästhesie- bzw. Atmungsgasen zur Gasmischung über eine Leitung derart steuert, daß dem Patienten eine gewünschte Gaskonzentration dem Patienten zugeführt wird. Auf diese Weise wird immer automatisch eine Regelung der gewünschten Gaskonzentration erzielt, die dem Patienten zugeführt wird.

Weitere Vorteile und Einzelheiten ergeben sich aus den Unteransprüchen.

Die Erfindung ist nachfolgend anhand eines in den Zeichnungen dargestellten Ausführungsbeispiels näher erläutert.

Es zeigen:
- FIG 1: eine Ausführungsform der Anordnung nach der Erfindung, bei der die Anordnung an ein Beatmungsgerät angeschlossen ist und
- FIG 2 und 3: weitere Ausführungsformen der Anordnung nach der Erfindung.

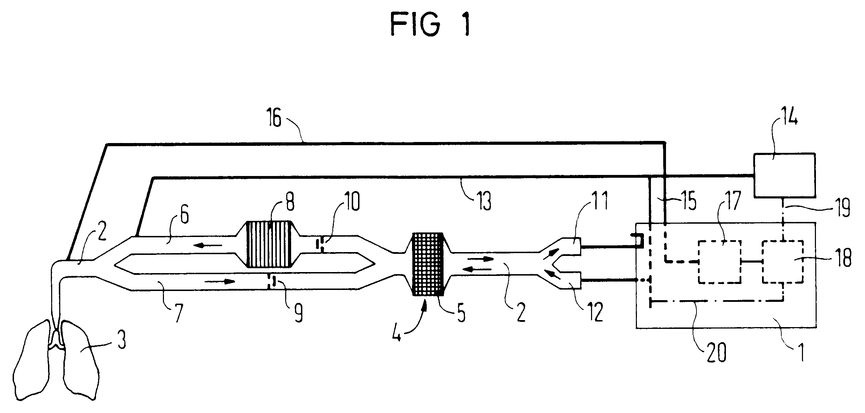

In der FIG 1 ist eine Anordnung zur Wiederverwendung von Anästhesiegasen bei der Inhalationsanästhesie gezeigt, die an ein Beatmungsgerät 1 angeschlossen ist. Die Anordnung umfaßt eine gemeinsame Leitung 2 für die Zufuhr und Abgabe von Anästhesie- und Atmungsgasen zu bzw. von einem Patienten 3. In der gemeinsamen Leitung 2 ist ein Adsorptionsfilter 4 angeordnet, das ein Adsorptionsmaterial 5 für die Adsorption und Desorption von Anästhesiegasen beinhaltet. Die Leitung zwischen dem Patienten 3 und dem Adsortionsfilter 4 ist teilweise in einen Einatmungszweig 6 und in einen Ausatmungszweig 7 aufgeteilt, wobei in dem Einatmungszweig 6 ein Absorber für Kohlendioxyd angeordnet ist. In dem Ein-und Ausatmungszweig 6,7 ist jeweils ein Einwegeventil 9 bzw. 10 angeordnet. Das Einwegeventil 10 des Einatmungszweiges 6 ist zwischen dem Adsorbtionsfilter 4 und dem Absorber 8 angebracht. Das freie Ende der gemeinsamen Leitung 2 bildet einen Y-förmigen Abschluß, dessen einen Zweig 11 über ein Beatmungsgerät 1 mit einem nicht gezeigten Evakuierungssystem für das Ausatmungsgas und dessen anderen Zweig 12 mit dem Beatmungsgerät 1 für die Zufuhr von Atmungsgas verbunden ist. An dem Einatmungszweig 6 zwischen dem Absorber 8 und dem Patienten 3 ist eine Leitung 13 für die Zufuhr von Anästhesiegasen aus einem Narkosemittelvergaser 14 angeschlossen. Der Narkosemittelvergaser 14 kann auch im Beatmungsgerät 1 angebracht sein. An der Leitung 13 ist eine Leitung 15 angeschlossen, die mit dem Beatmungsgerät 1 verbunden ist, das über diese Leitungen 15 und 13 bei Bedarf dem Patienten 3 frisches Atmungsgas zuführen kann.

Zwischen der Leitung 13 für die Zufuhr von Anästhesiegasen bzw. frischen Atemgasen und dem Patienten 3 ist an der Leitung 2 eine Gasleitung 16 angeschlossen, die eine Probe von der Gasmischung, die dem Patienten 3 zugeführt wird, an eine Vorrichtung 17 für die Messung der Gaskonzentration leitet. Ein Sensorsignal entsprechend der Gaskonzentration der Gasmischung wird danach einer Steuervorrichtung 18 zugeführt, die Feedback-kontrolliert die Zufuhr von Anästhesie- bzw. Atmungsgasen derart steuert, daß die gewünschte Gaskonzentration dem Patienten 3 zugeführt wird. Die strichpunktierte Linien 19,20 stellen Verbindungen zwischen der Steuervorrichtung 18 und dem Narkosemittelvergaser 14 bzw. zwischen der Steuervorrichtung 18 und der Leitung 15 und dem Zweig 12 zur Abgabe von Atmungsgasen dar. Sowohl die Meß- als auch die Steuervorrichtung 17,18 sind in diesem Ausführungsbeispiel im Beatmungsgerät 1 angeordnet.

In einer Ausatmungsphase passiert das Ausatmungsgas vom Patienten 3 den Ausatmungszweig 7, das Einwegeventil 9 und das Adsorptionsfilter 4. Das in einer früheren Einatmungsphase zugeführte Anästhesiegas, das der Patient 3 nicht absorbiert hat, sowie Feuchtigkeit aus dem Ausatmungsgas und ein geringerer Teil von Kohlendioxyd werden nun im Adsorptionsfilter 4 adsorbiert, während der Rest des Ausatmungsgases die Leitung 2 und den Zweig 11 passiert über das Beatmungsgerät 1 in das Evakuierungssystem gelangt. In der Einatmungsphase passiert frisches Atmungsgas vom Beatmungsgerät 1 den Zweig 12, die Leitung 2 und das Adsorptionsfilter 4 wobei das adsorbierte Anästhesiegas sowie die Feuchtigkeit und das adsorbierte Kohlendioxyd desorbiert werden und sich mit dem frischen Atmungsgas mischen. Da das Einwegeventil 9 in dem Ausatmungszweig 7 in der Einatmungsphase sperrt, wird diese Gasmischung über den Einatmungszweig 6, das Einwegeventil 10 und über den Absorber 8, der die Reste des Kohlendioxyds in der Gasmischung absorbiert, geleitet. Bevor die Gasmischung den Patienten 3 erreicht, wird eine Probe dieser Mischung entnommen, die, wie bereits beschrieben, über die Gasleitung 16 zur Meßvorrichtung 17 im Beatmungsgerät 1 geführt wird. Die Meßsignale werden der Steuervorrichtung 18 zugeführt, die in Abhängigkeit davon Signale an den Narkosevergaser 14 und das Beatmungsgerät 1 abgibt, damit die richtigen Mengen und die gewünschte Mischung von Anästhesie- und Atmungsgasen dem Patienten zugeführt werden. Durch die Leitung 13 werden diese Gase der Gasmischung zugeführt, bevor sie den Patienten 3 erreichen. In der Aufwachphase des Patienten 3 wird die Zufuhr von Atmungsgas vom Beatmungsgerät 1 über die gemeinsame Leitung 2 und den Einatmungszweig 6 gesperrt. Auch der Narkosevergaser 14 wird ausgeschaltet, wodurch lediglich frisches Atmungsgas vom Beatmungsgerät 1 dem Patienten 3 über die Leitungen 15 und 13 zugeführt wird.

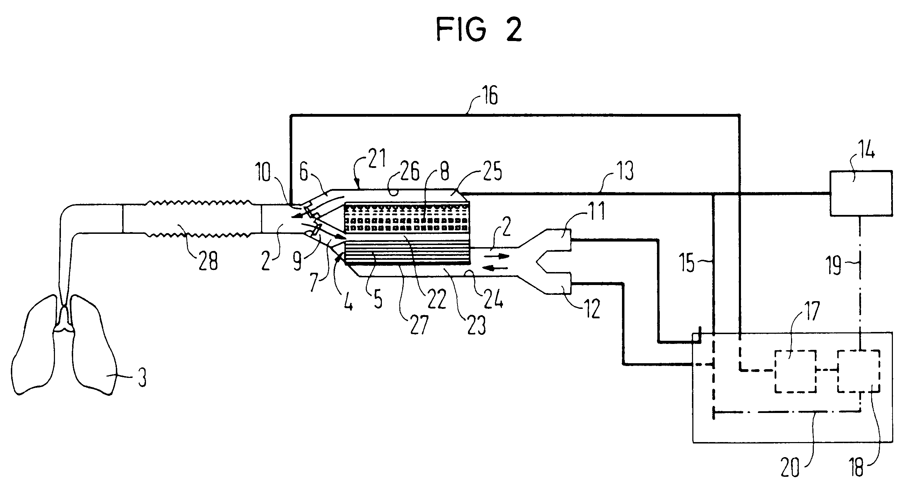

In der FIG 2 ist dargestellt, daß das Adsorptionsfilter 4 und der Absorber 8 parallel und mit Abstand zueinander in einem Behälter 21 derart angebracht sind, daß Kanäle gebildet werden. Ein erster Kanal 22 wird zwischen dem Adsorptionsfilter 4 und dem Absorber 8, ein zweiter Kanal 23 zwischen dem Adsorptionsfilter 4 und einer ersten Innenwand 24 und ein dritter Kanal 25 zwischen dem Absorber 8 und einer zweiten Innenwand 26 des Behälters 21 gebildet. Die eine Seite der Kanäle 22,23,25 ist offen und die anderen Seiten der Kanäle sind geschlossen. Der Kanal 22 ist an dem Ausatmungszweig 7, der Kanal 23 an der dem Patienten 3 abgewandten Seite des Behälters 21 an der gemeinsamen Leitung 2 und der Kanal 25 an dem Einatmungszweig 6 angeschlossen. Im Anschluß an das Adsorptionsfilter 4 an der dem Patienten 3 abgewandten Seite ist ein Keimfilter 27 angeordnet. Das Keimfilter 27 soll verhindern, daß Keime aus dem Ausatmungsgas die Schläuche und den Behälter kontaminieren, so daß sie nach der Behandlung nicht gereinigt werden müssen. An der Anordnung in der beschriebenen Ausführungsform ist wie im vorher beschriebenen Beispiel ein Narkosemittelvergaser 14 angeschlossen, der über die Leitung 13 z.B. an das geschlossene Ende des Kanals 25 für die Zufuhr von frischem Anästhesiegas angeschlossen ist. Es gibt außerdem eine Meßvorrichtung 17 die die Gaskonzentration des Einatmungsgases mißt und eine Steuervorrichtung 18 die wie bereits beschrieben das Volumen der Atmungs- bzw. Anästhesiegasen, die den Patienten 3 zugeführt werden, steuert.

In der Ausatmungsphase strömt das Ausatmungsgas durch einen kurzen flexiblen Schlauch 28, über die gemeinsame Leitung 2 und durch das Einwegeventil 9 im Ausatmungszweig 7. Das Ausatmungsgas strömt weiterhin durch den Kanal 22 und passiert danach das Adsorptionsfilter 4. Anästhesiegas und Feuchtigkeit aus der vorhergehenden Einatmungsphase, die der Patient 3 nicht absorbiert hat, sowie eine geringere Menge Kohlendioxyd werden in dem Adsorptionsfilter 4 adsorbiert, während der Rest des Ausatmungsgases durch die Leitung 2, den Zweig 11 und über das Beatmungsgerät 1 in das Evakuierungssystem heraustransportiert wird. In der Einatmungsphase wird frisches Atmungsgas vom Beatmungsgerät 1, den Zweig 12, die Leitung 2, den Keimfilter 27 und das Adsorptionsfilter 4 geleitet. Das adsorbierte Anästhesiegas und die Feuchtigkeit sowie das adsorbierte Kohlendioxyd werden desorbiert und zusammen mit dem Beatmungsgas dazu gebracht, den Kanal 22 und danach den Absorber 8 zu passieren, in dem die Kohlendioxydreste aufgefangen werden, um danach durch den Kanal 25, das Einwegeventil 10, die Leitung 2 und den Schlauch 28 zum Patient 3 zu strömen. Durch die beschriebene Ausführungsform der Anordnung können die Leitungen kurz sein, was den nicht zu vermeidenden "dead space" verkleinert.

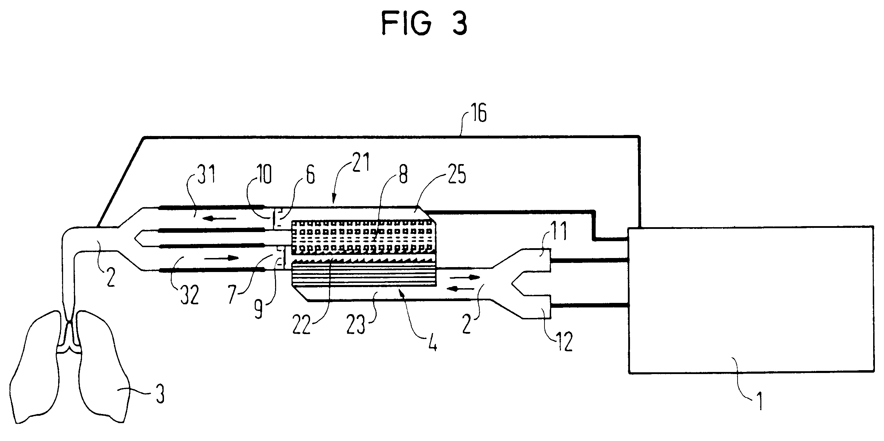

Wenn der Narkosearzt aus irgend einem Grund die Anordnung nicht in der Nähe des Patienten anbringen will, können die Ein- und Ausatmungszweige 6,7 mittels Patientenschläuche 31,32 verlängert werden. Diese Patientenschläuche 31,32 werden am Ende der gemeinsamen Leitung 2 angeschlossen, wie es in der FIG 3 dargestellt ist. Auf diese Weise wird die Anordnung genau so wirkungsvoll wie sie direkt am Patient 3 angeschlossen wäre wobei der "dead space" genau so klein gehalten werden kann wie der, der in Verbindung mit FIG 2 beschrieben wird.

Es hat sich gezeigt, daß als Adsorptionsmaterial 5 für das Adsorptionsfilter Kohle, insbesondere Kokosnußschalenkohle oder Holzkohle sehr geeignet ist. Die Kohle adsorbiert auch viel Feuchtigkeit aus dem Ausatmungsgas, das in der Einatmungsphase desorbiert und mit dem übrigen Einatmungsgas gemischt wird. Hierdurch ist eine sehr guter Feuchtigkeit-Wärmewechsler gegeben.

Der in den FIG 2 und 3 gezeigten Behälter, der das Adsorptionsmateral 5 und den Adsorber 8 enthält, ist aufgrund seiner geringen Größe und seiner Einfachheit in der Herstellung billig und ist daher als Wegwerfprodukt geeignet. Nach einer Behandlung wird der Behälter 21 von den Patientenschläuchen und den Leitungen getrennt und durch einen neuen Behälter ersetzt.

### Bezugszeichenliste

- 1: Beatmungsgerät
- 2: Leitung
- 3: Patient
- 4: Adsorptionsfilter
- 5: Adsorptionsmaterial
- 6: Einatmungszweig
- 7: Ausatmungszweig
- 8: Absorber
- 9,10: Einwegeventil
- 11,12: Zweig
- 13,15: Leitung
- 14: Narkosemittelvergaser
- 16: Gasleitung
- 17: Vorrichtung, Meßvorrichtung
- 18: Steuervorrichtung
- 19,20: strichpunktierte Linie
- 21: Behälter
- 22,23,25: Kanal
- 24,26: Innenwand
- 27: Keimfilter
- 28: Flexibler Schlauch
- 31,32: Patientenschlauch

## Patentansprüche

1. Verfahren zur Wiederverwendung von Anästhesiegasen bei der Inhalationsanästhesie, bei dem ein Patient (3) mittels Anästhesie- und/oder Beatmungsgas beatmet wird, wobei das Ausatmungsgas des Patienten (3) ein Adsorptionsfilter (4) für Anästhesiegas durchströmt und das nicht vom Patienten (3) absorbierte Anästhesiegas von einem im Adsorptionsfilter (4) vorhanden Adsorptionsmaterial (5) adsorbiert wird, während das restliche Ausatmungsgas dieses Filter (4) passiert und wobei bei einer Einatmung frisches Atmungsgas durch das Adsorptionsfilter (4) strömt, wobei das adsorbierte Anästhesiegas desorbiert und mit dem neuen Atmungsgas eine Gasmischung bildet, die dem Patienten (3) zugeführt wird, und wobei frisches Atmungsgas und/oder frisches Anästhesiegas, das das Adsorptionsfilter (4) nicht passiert, der Gasmischung direkt zugeführt werden kann, um die gewünschte Gaskonzentration aufrechtzuerhalten, **dadurch gekennzeichnet,** daß wenigstens das Gas, das das Adsorptionsfilter (4) durchsströmt, einen Absorber (8) für Kohlendioxyd passiert, bevor es dem Patienten (3) zugeführt wird.

2. Verfahren nach Anspruch 1, **dadurch gekennzeichnet,** daß die Gaskonzentration einer gewünschten Gassorte zwischen dem Patienten (3) und dem Absorber (8) gemessen und zur Steuerung des frischen Atmungs- bzw. Anästhesiegases, das der Gasmischung zugeführt wird, benutzt wird.

3. Verfahren nach Anspruch 2, **dadurch gekennzeichnet,** daß die Steuerung Feedback-kontrolliert erfolgt, wobei die gemessene Gaskonzentration den Istwert bildet und die gewünschte Gaskonzentration den Sollwert bestimmt.

4. Verfahren nach einem der Ansprüche 1 bis 3, **dadurch gekennzeichnet,** daß als Adsorptionsmaterial (5) Kohle verwendet wird.

5. Anordnung zur Durchführung des Verfahrens zur Wiederverwendung von Anästhesiegasen bei der Inhalationsanästhesie nach einem der Ansprüche 1 bis 4, die eine gemeinsame Leitung (2) für die Zufuhr und Abgabe von Anästhesie- und Atmungsgasen zu bzw. von einem Patienten (3) umfaßt, wobei in dieser Leitung (2) ein Adsorptionsfilter (4) angeordnet ist, das ein Adsorptionsmaterial (5) für die Adsorption und Desorption von Anästhesiegasen beinhaltet, und die eine zweite Leitung (13,15) für die Zufuhr von Anästhesie- und/oder Atmungsgasen zum Patienten (3) vorbei an dem Adsorptionsfilter (4) umfaßt, **dadurch gekennzeichnet,** daß die gemeinsame Leitung (2) zwischen dem Patienten (3) und dem Adsorptionsfilter (4) mindestens teilweise in einen Ein- bzw. Ausatmungszweig (6,7) aufgeteit ist, wobei in dem Einatmungszweig (6) ein Absorber (8) für Kohlendioxyd angeordnet ist.

6. Anordnung nach Anspruch 5, **dadurch gekennzeichnet,** daß als Adsorptionsmaterial (5) Kohle verwendet wird.

7. Anordnung nach Anspruch 6, **dadurch gekennzeichnet,** daß als Adsorptionsmaterial (5) Kokosnußschalenkohle verwendet wird.

8. Anordnung nach Anspruch 6, **dadurch gekennzeichnet,** daß als Adsorptionsmaterial (5) Holzkohle verwendet wird.

9. Anordnung nach einem der Ansprüche 5 bis 8, **dadurch gekennzeichnet,** daß das Adsorptionsfilter (4) und der Absorber (8) hauptsächlich paralell und mit Abstand zueinander derart in einem Behälter (21) angebracht sind, daß ein erster Kanal (22) zwischen dem Adsorptionsfilter (4) und dem Absorber (8) gebildet ist, daß ein zweiter Kanal (23) zwischen dem Adsorptionsfilter (4) und einer ersten Innenwand (24) des Behälters (21) gebildet ist und daß ein dritter Kanal (25) zwischen dem Absorber (8) und einer zweiten Innenwand (26) des Behälters (21) gebildet ist, daß der erste Kanal (22) an dem Ausatmungszweig (7) der zweite Kanal (23) an der dem Patienten (3) abgewandten Seite des Adsorptionsfilters (4) an der gemeinsamen Leitung (2) und der dritte Kanal (25) an dem Einatmungszweig (6) angeschlossen ist.

10. Anordnung nach einem der Ansprüche 5 bis 9, **dadurch gekennzeichnet,** daß im Anschluß an das Adsorptionsfilter (4) an der dem Patienten (3) abgewandten Seite ein Keimfilter (27) angeordnet ist.

11. Anordnung nach einem der Ansprüche 5 bis 10, **dadurch gekennzeichnet,** daß an der gemeinsamen Leitung (2) bzw. an dem Einatmungszweig (6) zwischen dem Patienten (3) und dem Absorber (8) eine Gasleitung (16) angeschlossen ist, die einen Teil der Gasmischung, die dem Patienten (3) zugeführt wird, zu einer Vorrichtung (17) zur Messung einer Gaskonzentration mindestens einer Gassorte führt, und wobei ein der Gaskonzentration entsprechendes Sensorsignal einer Steuervorrichtung (18) zugeführt wird, die Feedback-kontrolliert die Zufuhr von Anästhesie- bzw. Atmungsgasen zur Gasmischung über eine Leitung (13) derart steuert, daß dem Patienten (3) eine gewünschte Gaskonzentration zugführt wird.

12. Anordnung nach Anspruch 11, **dadurch gekennzeichnet,** daß die Meß- und Steuervorrichtungen in einem Beatmungsgerät angebracht sind.

## Claims

1. Method for the reuse of anaesthetic gases in inhalation anaesthesia, in which a patient (3) is ventilated using anaesthetic and/or ventilation gas, where the gas exhaled by the patient (3) flows through an adsorbent filter (4) for anaesthetic gas, and the anaesthetic gas not absorbed by the patient (3) is adsorbed by an adsorbent material (5) present in the adsorbent filter (4) while the remaining exhaled gas passes through this filter (4), and where on inhalation fresh respiratory gas flows through the adsorbent filter (4), where the adsorbed anaesthetic gas is desorbed and forms with the new respiratory gas a gas mixture which is passed to the patient (3), and where fresh respiratory gas and/or fresh anaesthetic gas, which does not pass through the adsorbent filter (4), can be fed directly to the gas mixture in order to maintain the desired gas concentration, characterized in that at least the gas which flows through the adsorbent filter (4) passes through an absorber (8) for carbon dioxide before it is passed to the patient (3).

2. Method according to Claim 1, characterized in that the gas concentration of a desired type of gas is measured between the patient (3) and the absorber (8) and used to control the fresh respiratory or anaesthetic gas fed to the gas mixture.

3. Method according to Claim 2, characterized in that the control takes place with feedback control, with the measured gas concentration forming the actual value and the desired gas concentration determining the specified value.

4. Method according to any of Claims 1 to 3, characterized in that charcoal is used as adsorbent material (5).

5. Arrangement for carrying out the method for the reuse of anaesthetic gases in inhalation anaesthesia according to any of Claims 1 to 4, which comprises a common line (2) for the supply and discharge of anaesthetic and respiratory gases to and from, respectively, a patient (3), where an adsorbent filter (4) is arranged in this line (2) and contains an adsorbent material (5) for the adsorption and desorption of anaesthetic gases, and which comprises a second line (13, 15) for the supply of anaesthetic and/or respiratory gases to the patient (3) by-passing the adsorbent filter (4), characterized in that the common line (2) between the patient (3) and the adsorbent filter (4) is at least partly divided into an inhalation and exhalation branch (6, 7), where an absorber (8) for carbon dioxide is arranged in the inhalation branch (6).

6. Arrangement according to Claim 5, characterized in that charcoal is used as adsorbent material (5).

7. Arrangement according to Claim 6, characterized in that coconut shell charcoal is used as adsorbent material (5).

8. Arrangement according to Claim 6, characterized in that wood charcoal is used as adsorbent material (5).

9. Arrangement according to any of Claims 5 to 8, characterized in that the adsorbent filter (4) and absorber (8) are installed, mainly parallel and spaced apart, in a container (21) in such a way that a first channel (22) is formed between the adsorbent filter (4) and the absorber (8), that a second channel (23) is formed between the adsorbent filter (4) and a first interior wall (24) of the container (21), and that a third channel (25) is formed between the absorber (8) and a second interior wall (26) of the container (21), that the first channel (22) is connected to the exhalation branch (7), the second channel (23) is connected to the side, which faces away from the patient (3), of the adsorbent filter (4) on the common line (2) and the third channel (25) is connected to the inhalation branch (6).

10. Arrangement according to any of Claims 5 to 9, characterized in that a bacterial filter (27) is arranged connected to the adsorbent filter (4) on the side facing away from the patient (3).

11. Arrangement according to any of Claims 5 to 10, characterized in that a gas line (16) is connected to the common line (2) and to the inhalation branch (6) between the patient (3) and the absorber (8) and passes a part of the gas mixture which is passed to the patient (3) to a device (17) to measure a gas concentration of at least one type of gas, and where a sensor signal corresponding to the gas concentration is fed to a control device (18) which controls with feedback control the supply of anaesthetic and respiratory gases to the gas mixture via a line (13) in such a way that a desired gas concentration is passed to the patient (3).

12. Arrangement according to Claim 11, characterized in that the measurement and control devices are installed in a ventilator.

## Revendications

1. Procédé pour réutiliser des gaz anesthésiques lors de l'anesthésie par inhalation, selon lequel on fait respirer au patient (3) un gaz anesthésique et/ou un gaz respiratoire, et selon lequel le gaz expiré par le patient (3) traverse un filtre d'adsorption (4) servant à adsorber le gaz anesthésique, et le gaz anesthésique absorbé par le patient (3) est adsorbé par un matériau d'adsorption (5) présent dans le filtre d'adsorption (4), tandis que le reste du gaz expiré traverse ce filtre (4) et selon lequel, lors d'une respiration, un gaz respiratoire frais traverse le filtre d'adsorption (4), et selon lequel le gaz anesthésique adsorbé est désorbé et forme avec le nouveau gaz respiratoire un mélange de gaz, qui est envoyé au patient (3), et selon lequel un gaz respiratoire frais et/ou un gaz anesthésique frais, qui n'a pas traversé le filtre d'adsorption (4), peut être envoyé directement au mélange gazeux, afin de maintenir la concentration désirée de gaz, caractérisé par le fait qu'au moins le gaz qui traverse le filtre d'adsorption (4), traverse un absorbeur (8) servant à absorber le gaz carbonique, avant son envoi au patient (3).

2. Procédé suivant la revendication 1, caractérisé par le fait que la concentration d'un type de gaz désiré est mesurée entre le patient (3) et l'absorbeur (8) et est utilisée pour commander le gaz respiratoire frais ou le gaz anesthésique frais, qui est introduit dans le mélange gazeux.

3. Procédé suivant la revendication 2, caractérisé par le fait que la commande s'effectue au moyen d'un contrôle par réaction, la concentration de gaz mesurée formant la valeur réelle et la concentration de gaz désirée formant la valeur de consigne.

4. Procédé suivant l'une des revendications 1 à 3, caractérisé par le fait qu'on utilise du carbone comme matériau adsorbant.

5. Dispositif pour la mise en oeuvre du procédé pour réutiliser des gaz anesthésiques lors de l'anesthésie par inhalation suivant l'une des revendications 1 à 4, qui comprend une conduite commune (2) pour amener et évacuer le gaz anesthésique et le gaz respiratoire en direction ou à partir d'un patient (3), un filtre d'adsorption (4), qui contient un matériau d'adsorption (5) pour réaliser l'adsorption et la désorption de gaz anesthésiques, étant disposé dans cette conduite (2), et qui comprend une seconde conduite (13,15) pour l'envoi d'un gaz anesthésique et/ou d'un gaz respiratoire au patient (3), qui est disposée à côté du filtre d'adsorption (4), caractérisé par le fait que la conduite commune (2) est subdivisée, entre le patient (3) et le filtre d'adsorption (4), au moins en partie en une branche d'aspiration et en une branche d'expiration (6,7), un absorbeur (8) servant à absorber le gaz carbonique étant disposé dans la branche d'aspiration (6).

6. Dispositif suivant la revendication 5, caractérisé par le fait qu'on utilise du carbone comme matériau d'absorption (5).

7. Dispositif suivant la revendication 6, caractérisé par le fait qu'on utilise du carbone sous la forme de coques de noix de coco en tant que matériau d'absorption (5).

8. Dispositif suivant la revendication 6, caractérisé par le fait qu'on utilise du charbon de bois comme matériau d'adsorption (5).

9. Dispositif suivant l'une des revendications 5 à 8, caractérisé par le fait que le filtre d'adsorption (4) et l'absorbeur (8) sont disposés essentiellement en parallèle et à une distance réciproque telle, dans un récipient (21), qu'un premier canal (22) est formé entre le filtre d'adsorption (4) et l'absorbeur (8), qu'un second canal (23) est formé entre le filtre d'adsorption (4) et une première paroi intérieure (24) du récipient (21), et qu'un troisième canal (25) est formé entre l'adsorbeur (8) et une seconde paroi intérieure (26) du récipient (21), que le premier canal (22) est raccordé à la branche d'expiration (7), que le second canal (23) est raccordé sur le côté, situé à l'opposé du patient (3), du filtre d'adsorption (4), à la conduite commune (2), et que le troisième canal (25) est raccordé à la branche d'aspiration (6).

10. Dispositif suivant l'une des revendications 5 à 9, caractérisé par le fait qu'un filtre (27) d'élimination des germes est disposé en aval du filtre d'adsorption (4), sur le côté éloigné du patient (3).

11. Dispositif suivant l'une des revendications 5 à 10, caractérisé par le fait qu'à la conduite commune (2) ou qu'à la branche d'aspiration (6) est raccordée, entre le patient (3) et l'absorbeur (8), une conduite de gaz (16), qui envoie une partie du mélange gazeux, qui est envoyé au patient (3), à un dispositif (17) servant à mesurer une concentration de gaz d'au moins un type de gaz, alors qu'un signal de détection, qui correspond à la concentration de gaz, est envoyé à un dispositif de commande (18), qui commande, d'une manière contrôlée par réaction, l'envoi de gaz anesthésique ou de gaz respiratoire au mélange gazeux par l'intermédiaire d'une conduite (13) de telle sorte qu'une concentration désirée de gaz est envoyée au patient (3).

12. Dispositif suivant la revendication 11, caractérisé par le fait que les dispositifs de mesure et de commande sont disposés dans un appareil respiratoire.
